# EUROPEAN PATENT SPECIFICATION

(11) **EP 2 872 032 B1**
(45) Date of publication and mention of the grant of the patent: **08.06.2016**
(21) Application number: 13742292.9
(22) Date of filing: 09.07.2013
(51) Int. Cl.: A61B 5/00, A61B 5/11

(54) **REGISTERING OF PHYSIOLOGICAL PARAMETERS BASED ON IMAGE ANALYSIS OF LIGHT REFLECTION**
AUFZEICHNUNG VON PHYSIOLOGISCHEN PARAMETERN AUF BASIS DER BILDANALYSE VON LICHTREFLEXION
ENREGISTREMENT DE PARAMÈTRES PHYSIOLOGIQUES BASÉ SUR L'ANALYSE D'IMAGES DE RÉFLEXION DE LUMIÈRE

(30) Priority: 10.07.2012 SE 1250807; 10.07.2012 US 201261669736 P
(43) Date of publication of application: 20.05.2015
(73) Proprietor: DeLaval Holding AB, 147 21 Tumba (SE)
(72) Inventor: KRIEF, Haim, Hadera (IL)
(74) Representative: Lilliehorn, Tobias
(86) International application number: PCT/SE2013/050880
(87) International publication number: WO 2014/011107

(56) References cited:
- EP-A1- 2 040 061
- EP-A2- 0 641 542
- WO-A2-2004/006748
- BY-C1- 11 990
- CN-C- 100 382 748
- GB-A- 2 437 250
- GB-A- 2 437 250
- US-A- 4 836 778
- US-A1- 2009 315 989
- US-A1- 2011 125 063
- US-B1- 7 740 588

## Description

### THE BACKGROUND OF THE INVENTION AND PRIOR ART

The present invention relates generally to solutions for determining physiological parameters of an animal. More particularly the invention relates to an apparatus according to the preamble of claim 1, a neckband according to claim 5 and a method according to the preamble of claim 7. The invention also relates to a computer program according to claim 11 and a computer readable medium according to claim 12.

To accomplish an efficient and animal friendly livestock handling it is important that the animals' physiological state and health condition be monitored. Of course, to this aim, regular farmer's inspections and veterinary examinations can never be excluded. However, as a complement thereto and to provide an ongoing supervision, various automatic systems can be employed. Today, a so-called activity meter may be employed, which is arranged in a neckband on the animal in order to register and report the animal's activity level to a remote location, e.g. via radio. The activity level is indicative of whether or not the animal is healthy. Nevertheless, the activity level is a very crude measure. Therefore, for ruminating animals such as cows, chewing behavior, rumen activity, cardiac activity and respiration would provide more valuable data. Known sensors for measuring these parameters are described in GB 2437250.

US 2010/0226543 discloses solutions for imaging objects, for example living body parts, wherein the objects are illuminated from a distance and surface vibrations thereof are image analyzed to extract various data, e.g. representing speech or heart beats. Document US 2011/125063 discloses an acoustic sensor configured to measure the acoustic energy of food ingestion, e.g. chewing sounds. Document US 4836778 discloses a motion analysis system to study movements of the jaw of a person.

An apparatus and a method for analyzing the human heart rate is disclosed in CN 10038278C.

### SUMMARY OF THE INVENTION

The object of the present invention is to offer a reliable, robust and animal-friendly solution for registering signals indicative of physiological parameters of a given animal.

The invention is defined in the independent claims 1, 7, 11, 12. Preferred embodiments are defined in the dependent claims.

According to one aspect of the invention, the object is achieved by the initially described apparatus, wherein the apparatus is configured to be arranged on the animal such that the outer side of the flexible wall contacts a neck region of the animal. The data processing means is specifically configured to derive a primary signal representing a chewing related parameter, which primary signal is caused by a vibration of the external body surface originating from internal organs in the animal. This primary signal can be used to draw conclusions concerning the animal's chewing behavior.
This apparatus is advantageous because it enables registering of highly complex signals in a very uncomplicated and straightforward manner. Thereby, important information concerning the animal's physiological status is obtainable, for instance related to food intake and digestion. Preferably, the data processing means is configured to derive at least one secondary signal representing: rumen activity, a cardiogram, heart rate, respiration rate, and/or a general activity level of the animal.
According to another preferred embodiment of this aspect of the invention, the light source includes a green laser. Green laser light is associated with a relatively low penetration, and therefore a large proportion of such light will be reflected back from the light reflective surface of the flexible wall, which is equivalent to a high degree of efficiency in terms of energy used.
According to a yet another preferred embodiment of this aspect of the invention, the data processing means is configured to determine a strongest light reflection in each image registered by an image sensor in the image registering means. From a set of such strongest light reflections determined in a series of images, the data processing means is further configured to derive the at least one signal. Hence, an uncomplicated time-varying signal is produced, which may serve as a basis for further analysis.

According to another preferred embodiment of this aspect of the invention, the object is achieved by a neckband adapted to be carried around the neck of a cow. The neckband includes the above-proposed apparatus and a fitting member configured to grip around the back of the neck of the cow, so as to reduce rotation movements of the neckband relative to the cow's neck. Thus, the apparatus is held in a relatively fix position without requiring a high pressure against the animal, which is advantageous from an animal-comfort point-of-view.
According to one preferred embodiment of this aspect of the invention, the neckband includes a weight member arranged essentially opposite to the fitting member on the neckband. The weight member is configured to pull the fitting member towards the back of the neck of the cow, and the proposed apparatus is arranged between the fitting member and the weight member. As a result, when the neckband is carried around the neck of a cow, the flexible wall will contact an external body surface of the animal while the risk of rotation movements of the neckband relative to the cow's neck is held relatively low and the flexible wall exerts a relatively low pressure on the external body surface. Of course, the weight member, in turn, may contain useful components, such a transponder and/or units for radio communication.
According to another aspect of the invention, the object is achieved by the method described initially, which presumes the use of an apparatus that has a housing including a flexible wall with an outer side and an inner side that is light reflective. The method involves arranging the apparatus so that the outer side of the flexible wall contacts a neck region of the animal. The at least one signal comprises a primary signal representing a chewing related parameter, which primary signal is caused by a vibration of the external body surface originating from internal organs in the animal. Said primary signal is used to draw conclusions concerning the animal's chewing behavior.

According to a further aspect of the invention the object is achieved by a computer program, which is directly loadable into the memory of a computer, and includes software adapted to implement the method proposed above when said program is run on a computer.

According to another aspect of the invention the object is achieved by a computer readable medium, having a program recorded thereon, where the program is to control a computer to perform the method proposed above when the program is loaded into the computer.

Further advantages, beneficial features and applications of the present invention will be apparent from the following description and the dependent claims.

### BRIEF DESCRIPTION OF THE DRAWINGS

The invention is now to be explained more closely by means of preferred embodiments, which are disclosed as examples, and with reference to the attached drawings.
- Figure 1: shows a ruminant animal carrying a neckband containing the proposed apparatus;
- Figure 2: illustrates an apparatus according to one embodiment of the invention that is arranged on an animal;
- Figure 3: shows an image sensor according to one embodiment of the invention illustrating an example of a set of strongest light reflections registered in a set of images;
- Figure 4: shows an example of a time-varying function derived from the set of strongest light reflections in a set of images in Figure 3; and
- Figure 5: illustrates, by means of a flow diagram, the general method according to the invention.

### DESCRIPTION OF PREFERRED EMBODIMENTS OF THE INVENTION

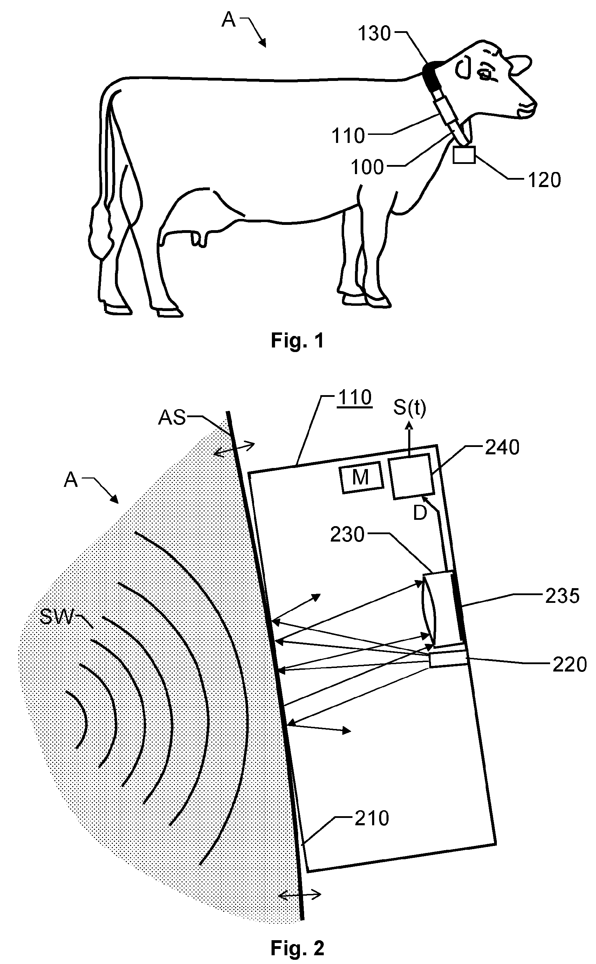

We refer initially to Figure 1, which shows a ruminant animal A carrying a neckband 100 containing the proposed apparatus 110 around its neck. The neckband 100 has a fitting member 130 configured to grip around the back of the cow's neck, so as to reduce rotation movements of the neckband 100 relative to the cow's neck. The fitting member 130 may thus include a section (e.g. of plastic) that is pre-shaped to fit the general contour of the back of the cow's neck.
According to one preferred embodiment of the invention, the neckband 100 includes a weight member 120, which is arranged essentially opposite to the fitting member 130 on the neckband 100, and is configured to pull the fitting member 130 towards the back of the neck of the cow. The apparatus 110 is arranged between the fitting member 130 and the weight member 120, such that when the neckband 100 is carried around the neck of a cow a sensor part of the apparatus 110 is drawn towards and to contact an external body surface of the animal A so that the sensor part of the apparatus 110 contacts the external body surface. At the same time, the risk that the neckband 100 rotates relative to the cow's neck is held relatively low, and said sensor part exerts a relatively low pressure on the animal's A body. The weight member 120 preferably contains high-density components, such as batteries and/or radio components, e.g. for transmitting any signals S(t) produced by the apparatus 110.

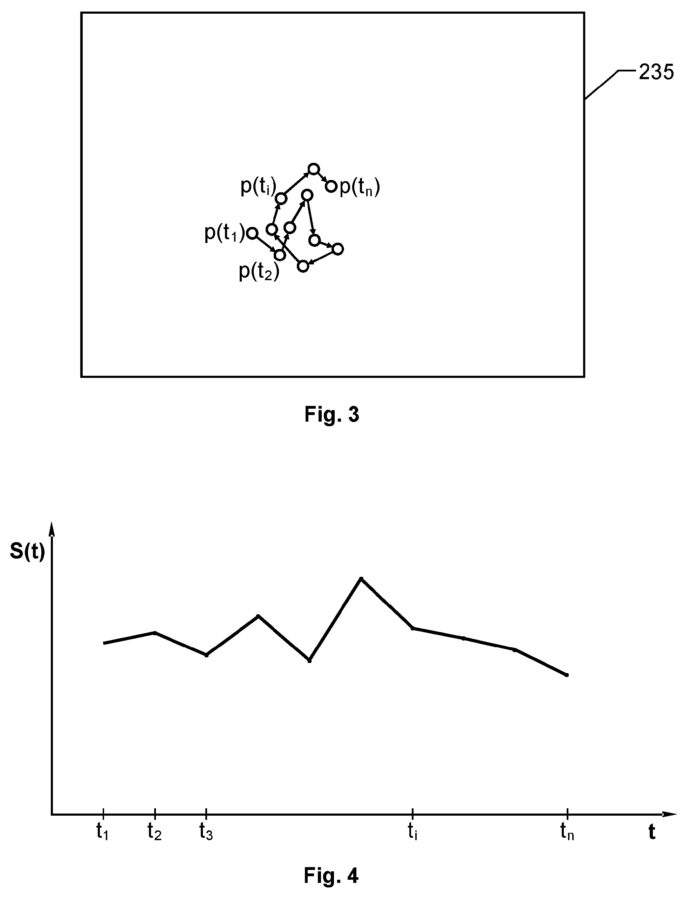

Naturally, in addition to the proposed apparatus 110 and any weight member 120, the neckband 100 may include one or more other types of sensors, preferably configured to register signals different from those registered by the apparatus 110.
Referring to Figure 2, more specifically, a flexible wall 210 of the apparatus 110 will exert a relatively low pressure on an external body surface AS of the animal A while the risk of rotation movements of the neckband 100 relative to the cow's neck is held relatively low. Figure 2 shows a side view of the apparatus 110 according to one embodiment of the invention when arranged on an animal A.
The apparatus 110 includes a housing, which, in turn, contains: a flexible wall 210, a light source 220, an image registering means 230 and a data processing means 240.
The flexible wall 210 represents the above-mentioned sensor part and has an outer side configured to contact the external body surface AS of the animal A. An inner side of the flexible wall 210 contains a light reflective surface towards an interior of the housing.
The light source 220 is configured to illuminate the light reflective surface. Preferably, the light source 220 contains a green laser because due to the relatively low degree of penetration of green laser light, such a light source is associated with comparatively high degree of energy efficiency.
The image registering means 230 is configured to capture image data D representing the light reflective surface. To this aim, the image registering means 230 includes an image sensor 235 (see Figure 3).
The data processing means 240 is configured to receive the image data D, and based thereon; produce at least one signal S(t) indicative of at least one physiological parameter of the animal A.

As mentioned above, the apparatus 110 is configured to be arranged on the animal A, such that the outer side of the flexible wall 210 contacts a neck region of the animal A. Thereby, any signals SW (typically sounds, i.e. mechanical waves) originating from internal organs in the animal A may propagate through the animal's A body and cause vibrations of the external body surface AS of the animal A. Consequently, by studying the vibrations of the external body surface AS, it is possible to draw conclusions concerning the animal's A chewing behavior, rumen activity, cardiac activity (cardiogram and/or heart rate) as well as respiration rate. In fact, a general activity level of the animal A may also be derived, since animal activity corresponds to low-frequency noise of high magnitude (relative to the other signals).

According to the invention, the data processing means 240 is configured to derive a primary signal S(t) representing a chewing related parameter. Additionally, the data processing means 240 may also be configured to derive at least one secondary signal S(t) representing: rumen activity, a cardiogram, heart rate, respiration rate and/or a general activity level of the animal A.

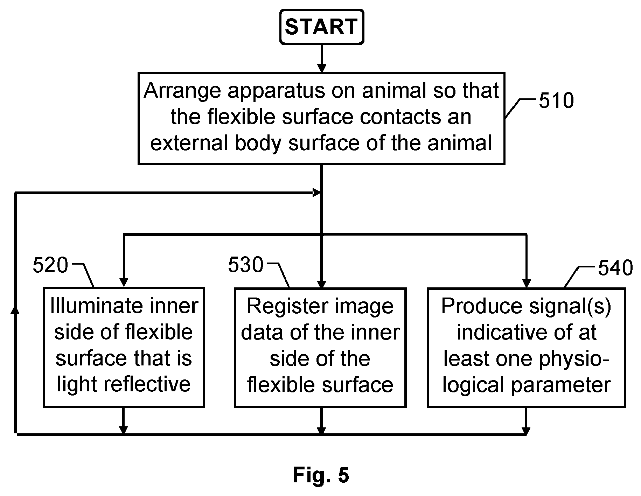

Figure 3 shows the image sensor 235 according to a preferred embodiment of the invention. Here, we see an example of a set of strongest light reflections p(t₁), p(t₂), ... p(tᵢ), ... p(tₙ) registered in a set of images, say n images. Thus, each image essentially results in a point in a two-dimensional plane, where the point represents the location of the strongest light reflection in that image. This, in turn, may be translated by the data processing means 240 into a one-dimensional function S(t) of time t as illustrated in Figure 4.
In Figure 4 we an example of a time-varying function S(t) derived from the set of strongest light reflections p(t₁), p(t₂), ... p(tᵢ), ... p(tₙ) in Figure 3, where the magnitude of S(t) is equivalent to a distance between two consecutive strongest light p(tᵢ). Preferably, the data processing means 240 is configured to apply a threshold, such that a strongest light reflection corresponding to an exceptionally large magnitude is discarded. Naturally, according to the invention, many alternative (and more complex) parameters may also be derived from the image data D captured by the image sensor 235.
The above procedure implemented by the processing means 240 is preferably controlled by a computer program M loaded into a memory of the processing means 240, or an external memory unit accessible by the processing means 240. The computer program, in turn, contains software for controlling the steps of the procedure when the program is run on the processing means 240. In order to sum up, we will now describe the general method according to the invention with reference to the flow diagram in Figure 5.

In a first step 510, an apparatus 110 is arranged on an animal A, so that an outer side of its flexible wall 210 contacts an external body surface AS, preferably in the neck region.

In a following step 520, the light reflective surface is illuminated from an interior of the apparatus' 110 housing. A step 530, parallel to step 520, registers image data D representing the reflective surface of the inner side of the flexible wall 210. A step 540, parallel to steps 520 and 530, processes the image data D to produce at least one signal S(t) indicative of at least one physiological parameter of the animal A. Then, the procedure loops back to steps 520, 530 and 540 for repeated measurement.

All of the process steps, as well as any sub-sequence of steps, described with reference to Figure 5 above may be controlled by means of a programmed computer apparatus. Moreover, although the embodiments of the invention described above with reference to the drawings comprise computer apparatus and processes performed in computer apparatus, the invention thus also extends to computer programs, particularly computer programs on or in a carrier, adapted for putting the invention into practice. The program may be in the form of source code, object code, a code intermediate source and object code such as in partially compiled form, or in any other form suitable for use in the implementation of the process according to the invention. The program may either be a part of an operating system, or be a separate application. The carrier may be any entity or device capable of carrying the program. For example, the carrier may comprise a storage medium, such as a Flash memory, a ROM (Read Only Memory), for example a DVD (Digital Video/Versatile Disk), a CD (Compact Disc) or a semiconductor ROM, an EP-ROM (Erasable Programmable Read-Only Memory), an EEPROM (Electrically Erasable Programmable Read-Only Memory), or a magnetic recording medium, for example a floppy disc or hard disc. Further, the carrier may be a transmissible carrier such as an electrical or optical signal which may be conveyed via electrical or optical cable or by radio or by other means. When the program is embodied in a signal which may be conveyed directly by a cable or other device or means, the carrier may be constituted by such cable or device or means. Alternatively, the carrier may be an integrated circuit in which the program is embedded, the integrated circuit being adapted for performing, or for use in the performance of, the relevant processes.

Although the invention has been described primarily with reference to cows, the invention is equally well adapted for any other kind of animals, such as goats, sheep or buffaloes.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

## Claims

1. An apparatus (110) for registering signals indicative of physiological parameters of an animal (A) based on illumination of a surface and analysis of light reflections from the surface, the apparatus including a housing comprising:
a flexible wall (210), an outer side of which is configured to contact an external body surface (AS) of the animal (A), the flexible wall (210) having an inner side containing a light reflective surface towards an interior of the housing;
a light source (220), configured to illuminate the light reflective surface;
an image registering means (230), configured to capture image data (D) representing the light reflective surface; and
a data processing means (240), configured to receive the image data (D) and based thereon produce at least one signal (S(t)) indicative of at least one physiological parameter of the animal (A),
**characterized in that** the apparatus is configured to be arranged on the animal (A) such that the outer side of the flexible wall (210) contacts a neck region of the animal (A), and the data processing means (240) is configured to derive a primary signal (S(t)) representing a chewing related parameter, which primary signal (S(t)) is caused by a vibration of the external body surface (AS) originating from internal organs in the animal (A), and allows to draw conclusions concerning the animal's (A) chewing behavior.

2. The apparatus (110) according to claim 1, wherein the data processing means (240) is configured to derive at least one secondary signal (S(t)) representing at least one of: rumen activity, a cardiogram, heart rate, respiration rate, and a general activity level of the animal (A).

3. The apparatus (110) according to claim 1, wherein the light source (220) comprises a green laser.

4. The apparatus (110) according to any one of the preceding claims, wherein the data processing means (240) is configured to determine a strongest light reflection ((p(t₁), ..., (p(tₙ)) in each image registered by an image sensor (235) in the image registering means (230), and derive the at least one signal (S(t)) from a set of strongest light reflections ((p(t₁), ..., (p(tₙ)) determined in a series of images.

5. A neckband (100) adapted to be carried around the neck of a cow, comprising the apparatus (110) according to any one of the preceding claims, and a fitting member (130) configured to grip around the back of the neck of the cow so as to reduce rotation movements of the neckband (100) relative to the cow's neck.

6. The neckband (100) according to claim 5, comprising a weight member (120) arranged essentially opposite to the fitting member (130) on the neckband (100), and configured to pull the fitting member (130) towards the back of the neck of the cow, the apparatus (110) being arranged between the fitting member (130) and the weight member (120) such that when the neckband is carried around the neck of a cow the flexible wall (210) contacts an external body surface (AS) of the animal (A) while a risk of rotation movements of the neckband (100) relative to the cow's neck is held relatively low and the flexible wall (210) exerts a relatively low pressure on the external body surface (AS).

7. A method for registering signals indicative of physiological parameters of an animal (A) based on illumination of a surface and analysis of light reflections from the surface, by using an apparatus (110) having a housing including a flexible wall (210) with an outer side and an inner side that is light reflective, said method comprising:
arranging the apparatus (110) such that the flexible wall (210) contacts an external body surface (AS) of the animal (A);
illuminating the light reflective surface from an interior of the housing;
capturing image data (D) representing the reflective surface; and
processing the image data (D) to produce at least one signal (S(t)) indicative of at least one physiological parameter of the animal (A),
**characterized by** the method further comprising:
arranging the apparatus (110) so that the outer side of the flexible wall (210) contacts a neck region of the animal (A), wherein the at least one signal (S(t)) comprises a primary signal representing a chewing related parameter, which primary signal (S(t)) is caused by a vibration of the external body surface (AS) originating from internal organs in the animal (A); and
using said primary signal (S(t)) to draw conclusions concerning the animal's (A) chewing behavior.

8. The method according to claim 7, comprising deriving at least one secondary signal (S(t)) representing at least one of:
rumen activity, a cardiogram, heart rate, respiration rate, and a general activity level of the animal (A).

9. The method according to claim 7 or 8, wherein the illuminating of the light reflective surface is carried out using a green laser (220).

10. The method according to any one of claims 7 to 9, comprising:
determining a strongest light reflection ((p(t₁), ..., (p(tₙ)) in each image registered by an image sensor (235) in the image registering means (230), and
deriving the at least one signal (S(t)) from a set of strongest light reflections ((p(t₁), ..., (p(tₙ)) determined in a series of images.

11. A computer program comprising software for controlling the steps of any of the claims 7 to 10 when said program is run on an apparatus according to claim 1.

12. A computer readable storage medium (M), having a program recorded thereon, where the program is to make an apparatus according to claim 1 carry out the steps of any of the claims 7 to 10 when the program is loaded into the apparatus.

## Patentansprüche

1. Vorrichtung (110) zum Registrieren von Signalen, die anzeigend sind für physiologische Parameter eines Tieres (A), auf Grundlage einer Beleuchtung einer Oberfläche und einer Analyse von Lichtreflexionen von der Oberfläche, wobei die Vorrichtung ein Gehäuse beinhaltet, umfassend:
eine flexible Wand (210), deren Außenseite ausgelegt ist, eine externe Körperoberfläche (AS) des Tieres (A) zu kontaktieren, wobei die flexible Wand (210) eine innere Seite aufweist, welche eine lichtreflektierende Oberfläche zu einem Inneren des Gehäuses hin enthält;
eine Lichtquelle (220), welche ausgelegt ist, die lichtreflektierende Oberfläche zu beleuchten;
eine Bilderfassungseinrichtung (230), welche ausgelegt ist, Bilddaten (D), die die lichtreflektierende Oberfläche repräsentieren, zu erfassen; und
eine Datenverarbeitungseinrichtung (240), welche ausgelegt ist, die Bilddaten (D) zu empfangen und darauf basierend zumindest ein Signal (S(t)) zu erzeugen, das anzeigend für zumindest einen physiologischen Parameter des Tieres (A) ist,
**dadurch gekennzeichnet, dass** die Vorrichtung ausgelegt ist, an dem Tier (A) derart angeordnet zu werden, dass die Außenseite der flexiblen Wand (210) einen Nackenbereich des Tieres (A) kontaktiert, und die Datenverarbeitungseinrichtung (240) ausgelegt ist, ein primäres Signal (S(t)), das einen kaubezogenen Parameter repräsentiert, abzuleiten, wobei das primäre Signal (S(t)) durch eine Vibration der externen Körperoberfläche (AS) hervorgerufen wird, welche von inneren Organen des Tieres (A) herrührt und es gestattet, Rückschlüsse auf das Kauverhalten des Tieres (A) zu ziehen.

2. Vorrichtung (110) nach Anspruch 1, wobei die Datenverarbeitungseinrichtung (240) ausgelegt ist, zumindest ein sekundäres Signal (S(t)) abzuleiten, welches zumindest eines von folgendem repräsentiert: eine Pansenaktivität, ein Kardiogramm, eine Herzrate, eine Atmungsrate und ein allgemeines Aktivitätsniveau des Tieres (A).

3. Vorrichtung (110) nach Anspruch 1, wobei die Lichtquelle (220) einen grünen Laser umfasst.

4. Vorrichtung (110) nach einem der vorhergehenden Ansprüche, wobei die Datenverarbeitungseinrichtung (240) ausgelegt ist, eine stärkste Lichtreflexion ((p(t₁), ... , (p(tₙ)) in jedem Bild, das durch einen Bildsensor (235) in der Bildregistrierungseinrichtung (230) registriert ist, zu bestimmen und das zumindest eine Signal (S(t)) aus einem Satz stärkster Lichtreflexionen ((p(t₁), ... , (p(tₙ)) abzuleiten, die in einer Reihe von Bildern bestimmt sind.

5. Nackenband (100), welches ausgelegt ist, um den Nacken einer Kuh herum getragen zu werden, umfassend die Vorrichtung (110) nach einem der vorhergehenden Ansprüche und ein Befestigungselement (130), welches ausgelegt ist, um die Rückseite des Nackens der Kuh herum zu greifen, um so Drehbewegungen des Nackenbands (100) relativ zu dem Nacken der Kuh zu verringern.

6. Nackenband (100) nach Anspruch 5, umfassend ein Gewichtselement (120), dass im wesentlichen gegenüberliegend zu dem Befestigungselement (130) an dem Nackenband (100) angeordnet ist und ausgelegt ist, das Befestigungselement (130) zu der Rückseite des Nackens der Kuh zu ziehen, wobei die Vorrichtung (110) zwischen dem Befestigungselement (130) und dem Gewichtselement (120) derart angeordnet ist, dass dann, wenn das Nackenband um den Nacken einer Kuh getragen wird, die flexible Wand (210) eine externe Körperoberfläche (AS) des Tieres (A) kontaktiert, während ein Risiko von Drehbewegungen des Nackenbands (100) relativ zu dem Nacken der Kuh relativ niedrig gehalten wird und die flexible Wand (210) einen relativ geringen Druck auf die externe Körperoberfläche (AS) ausübt.

7. Verfahren zum Registrieren von Signalen, die anzeigend sind für physiologische Parameter eines Tieres (A), auf Grundlage einer Beleuchtung einer Oberfläche und einer Analyse von Lichtreflexionen von der Oberfläche, unter Verwendung einer Vorrichtung (110), welche ein Gehäuse aufweist, das eine flexible Wand (210) mit einer äußeren Seite und einer inneren Seite, welche lichtreflektierend ist, beinhaltet, wobei das Verfahren umfasst:
Anordnen der Vorrichtung (110) derart, dass die flexible Wand (210) eine externe Körperoberfläche (AS) des Tieres (A) kontaktiert;
Beleuchten der lichtreflektierenden Oberfläche von einem Inneren des Gehäuses;
Aufnehmen von Bilddaten (D), welche die lichtreflektierende Oberfläche repräsentieren; und
Verarbeiten der Bilddaten (D), um zumindest ein Signal (S(t)) zu erzeugen, welches anzeigend für zumindest einen physiologischen Parameter des Tieres (A) ist,
**dadurch gekennzeichnet, dass** das Verfahren ferner umfasst:
Anordnen der Vorrichtung (110) so, dass die Außenseite der flexiblen Wand (210) einen Nackenbereich des Tieres (A) kontaktiert, wobei das zumindest eine Signal (S(t)) ein primäres Signal umfasst, das einen kaubezogenen Parameter repräsentiert, wobei das primäre Signal (S(t)) durch eine Vibration der externen Körperoberfläche (AS) hervorgerufen wird, welche von inneren Organen des Tieres (A) herrührt; und
Verwenden des primären Signals (S(t)), um Rückschlüsse auf das Kauverhalten des Tieres (A) zu ziehen.

8. Verfahren nach Anspruch 7, umfassend ein Ableiten zumindest eines sekundären Signals (S(t)), welches zumindest eines von folgendem repräsentiert: eine Pansenaktivität, ein Kardiogramm, eine Herzrate, eine Atmungsrate und ein allgemeines Aktivitätsniveau des Tieres (A).

9. Verfahren nach Anspruch 7 oder 8, wobei das Beleuchten der lichtreflektierenden Oberfläche unter Verwendung eines grünen Lasers (220) ausgeführt wird.

10. Verfahren nach einem der Ansprüche 7 bis 9, umfassend:
Bestimmen einer stärksten Lichtreflexion ((p(t₁), ... , (p(tₙ)) in jedem Bild, das durch einen Bildsensor (235) in der Bildregistrierungseinrichtung (230) registriert wird, und
Ableiten des zumindest einen Signals (S(t)) aus einem Satz stärkster Lichtreflexionen ((p(t₁), ..., (p(tₙ)), die in einer Reihe von Bildern bestimmt werden.

11. Computerprogramm, umfassend eine Software zum Steuern der Schritte von jedwedem der Ansprüche 7 bis 10, wenn das Programm auf einer Vorrichtung nach Anspruch 1 ausgeführt wird.

12. Computerlesbares Speichermedium (M), welches ein Programm darauf aufgezeichnet aufweist, wobei das Programm eine Vorrichtung nach Anspruch 1 dazu veranlasst, die Schritte nach jedwedem der Ansprüche 7 bis 10 auszuführen, wenn das Programm in die Vorrichtung geladen ist.

## Revendications

1. Appareil (110) destiné à l'enregistrement de signaux indicatifs des paramètres physiologiques d'un animal (A) basé sur l'éclairement d'une surface et l'analyse des réflexions de lumière provenant de la surface, l'appareil incluant un boîtier comprenant :
une paroi souple (210), dont un côté extérieur est configuré pour entrer en contact avec une surface de corps externe (AS) de l'animal (A), la paroi souple (210) ayant un côté intérieur contenant une surface réfléchissant la lumière en direction de l'intérieur du boîtier ;
une source lumineuse (220), configurée pour éclairer la surface réfléchissant la lumière ;
un moyen d'enregistrement d'image (230), configuré pour capturer des données d'image (D) représentant la surface réfléchissant la lumière ; et
un moyen de traitement de données (240), configuré pour recevoir les données d'image (D) et, basé sur celui-ci, produire au moins un signal (S(t)) indicatif d'au moins un paramètre physiologique de l'animal (A),
**caractérisé en ce que** l'appareil est configuré pour être disposé sur l'animal (A) de sorte que le côté extérieur de la paroi souple (210) entre en contact avec une zone du cou de l'animal (A), et le moyen de traitement de données (240) est configuré pour dériver un signal principal (S(t)) représentant un paramètre relatif à la mastication, lequel signal principal (S(t)) est provoqué par une vibration de la surface de corps externe (AS) ayant pour origine les organes internes de l'animal (A), et permet de tirer des conclusions concernant le comportement de mastication de l'animal (A).

2. Appareil (110) selon la revendication 1, dans lequel le moyen de traitement de données (240) est configuré pour dériver au moins un signal secondaire (S(t)) représentant au moins l'un des éléments parmi : une activité de rumination, un cardiogramme, la fréquence cardiaque, la fréquence respiratoire et un niveau d'activité général de l'animal (A).

3. Appareil (110) selon la revendication 1, dans lequel la source lumineuse (220) comprend un laser vert.

4. Appareil (110) selon l'une quelconque des revendications précédentes, dans lequel le moyen de traitement de données (240) est configuré pour déterminer une plus forte réflexion de lumière ((p(t1), ..., (p(tn)) dans chaque image enregistrée par un capteur d'image (235) dans le moyen d'enregistrement d'image (230), et dérive l'au moins un signal (S(t)) à partir d'un jeu des plus fortes réflexions de lumière ((p(t1), ..., (p(tn)) déterminé dans une série d'images.

5. Collier (100) adapté pour être porté autour du cou d'une vache, comprenant l'appareil (110) selon l'une quelconque des revendications précédentes, et un élément d'ajustage (130) configuré pour s'agripper autour de l'arrière du cou de la vache, de façon à réduire les mouvements de rotation du collier (100) par rapport au cou de la vache.

6. Collier (100) selon la revendication 5, comprenant un élément de poids (120) disposé essentiellement à l'opposé de l'élément d'ajustage (130) sur le collier (100), et configuré pour tirer l'élément d'ajustage (130) en direction de l'arrière du cou de la vache, l'appareil (110) étant disposé entre l'élément d'ajustage (130) et l'élément de poids (120) de sorte que lorsque le collier est porté autour du cou d'une vache, la paroi souple (210) entre en contact avec une surface de corps externe (AS) de l'animal (A) tandis qu'un risque de mouvements de rotation du collier (100) par rapport au cou de la vache est maintenu relativement faible, et la paroi souple (210) exerce une pression relativement faible sur la surface de corps externe (AS).

7. Procédé destiné à enregistrer des signaux indicatifs des paramètres physiologiques d'un animal (A) basé sur l'éclairement d'une surface et sur l'analyse des réflexions de lumière provenant de la surface, en utilisant un appareil (110) ayant un boîtier incluant une paroi souple (210) avec un côté extérieur et un côté intérieur qui sont réflecteurs de lumière, ledit procédé comprenant :
la disposition de l'appareil (110) de sorte que la paroi souple (210) entre en contact avec une surface de corps externe (AS) de l'animal (A) ;
l'éclairement de la surface réfléchissant la lumière à partir d'un intérieur du boîtier ;
la capture de données d'image (D) représentant la surface réflectrice ; et
le traitement des données d'image (D) pour produire au moins un signal (S(t)) indicateur d'au moins un paramètre physiologique de l'animal (A),
**caractérisé en ce que** le procédé comprend en outre :
la disposition de l'appareil (110) de sorte que le côté extérieur de la paroi souple (210) entre en contact avec une zone du cou de l'animal (A), dans lequel l'au moins un signal (S(t)) comprend un signal principal (S(t)) représentant un paramètre relatif à la mastication, lequel signal principal (S(t)) est provoqué par une vibration de la surface de corps externe (AS) ayant pour origine les organes internes de l'animal (A) ; et
l'utilisation dudit signal principal (S(t)) pour tirer des conclusions concernant le comportement de mastication de l'animal (A).

8. Procédé selon la revendication 7, comprenant la dérivation d'au moins un signal secondaire (S(t)) représentant au moins l'un des éléments parmi : une activité de rumination, un cardiogramme, la fréquence cardiaque, la fréquence respiratoire et un niveau d'activité général de l'animal (A).

9. Procédé selon la revendication 7 ou 8, dans lequel l'éclairement de la surface réfléchissant la lumière est généré à partir d'un laser vert (220).

10. Procédé selon l'une quelconque des revendications 7 à 9, comprenant :
la détermination d'une plus forte réflexion de lumière ((p(t1), ..., (p(tn)) dans chaque image enregistrée par un capteur d'image (235) dans le moyen d'enregistrement d'image (230), et
la dérivation de l'au moins un signal (S(t)) à partir d'un jeu des plus fortes réflexions de lumière ((p(t1), ..., (p(tn)) déterminé sur une série d'images.

11. Programme informatique, comprenant un logiciel destiné au contrôle des étapes de l'une quelconque des revendications 7 à 10 lorsque ledit programme est exécuté sur un appareil selon la revendication 1.

12. Support de stockage (M) lisible par un ordinateur, ayant un programme enregistré dessus, où le programme fait en sorte qu'un appareil selon la revendication 1 exécute les étapes de l'une quelconque des revendications 7 à 10 lorsque le programme est chargé dans l'appareil.
